Europäisches Patentamt

European Patent Office

Office européen des brevets

(11) Veröffentlichungsnummer: **0 266 444**
**A1**

(12) **EUROPÄISCHE PATENTANMELDUNG**

(21) Anmeldenummer: 86115237.9

(22) Anmeldetag: 04.11.86

(51) Int. Cl.⁴: **A61B 5/02**

(43) Veröffentlichungstag der Anmeldung:
**11.05.88 Patentblatt 88/19**

(84) Benannte Vertragsstaaten:
**AT BE CH DE ES FR GB GR IT LI LU NL SE**

(71) Anmelder: **von Zehmen, Thomas, Dipl.-Ing.**
**Crössmannstrasse 3**
**D-6360 Friedberg(DE)**

Anmelder: **Gelenk, Peter, Dipl.-Ing.**
**Donaustrasse 7**
**D-6450 Hanau(DE)**

(72) Erfinder: **von Zehmen, Thomas, Dipl.-Ing.**
**Crössmannstrasse 3**
**D-6360 Friedberg(DE)**
Erfinder: **Gelenk, Peter, Dipl.-Ing.**
**Donaustrasse 7**
**D-6450 Hanau(DE)**

(74) Vertreter: **Blumbach Weser Bergen Kramer**
**Zwirner Hoffmann Patentanwälte**
**Sonnenbergerstrasse 43**
**D-6200 Wiesbaden 1(DE)**

(54) **System zur Feststellung von Blutdruckpulsationen.**

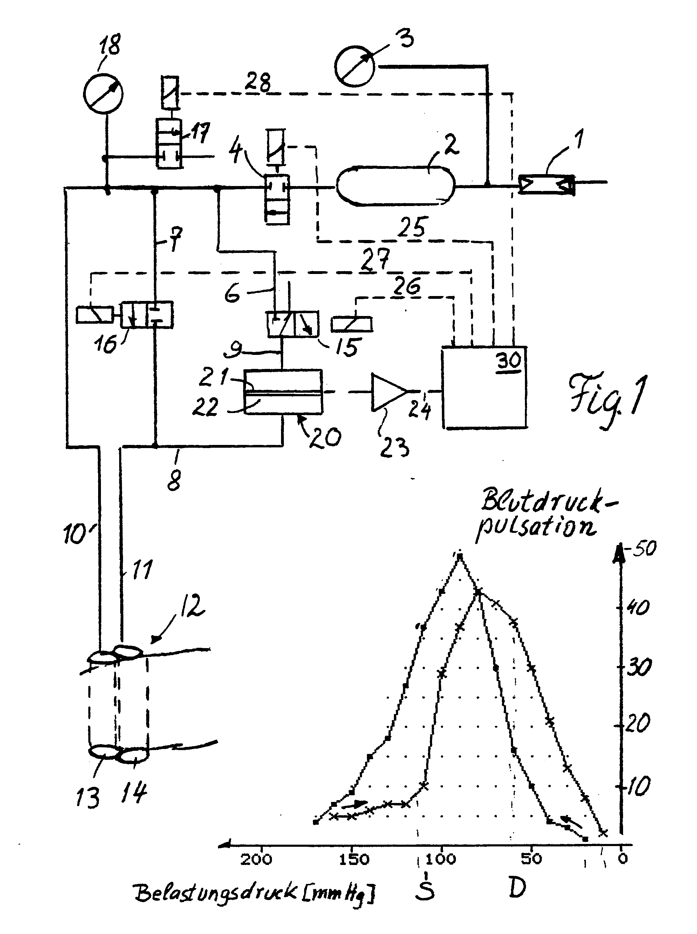

(57) System zur Feststellung von Blutdruckpulsationen mit einer Manschette (12), mit der eine Belastungskurve für die untersuchte Person erzeugt wird. Hierzu sind elektromagnetische Ventile (4, 15, 16) und eine Druckmeßeinrichtung (20) vorgesehen. Eine Steuerelektronik (30) steuert die Ventile derart, daß während jeder Druckstufe die Druckpulsationen in der Manschette (12) gemessen werden. Die Meßwerte werden gespeichert, zu Summen-, Differenz-und Verhältniswert-Tabellen verarbeitet und über Drucker oder Bildschirm ausgegeben. (Fig. 1).

Fig. 1

EP 0 266 444 A1

## System zur Feststellung von Blutdruckpulsationen

Die Erfindung bezieht sich auf ein System zur Feststellung von Blutdruckpulsationen, bei der eine Fluiddruckerzeugungseinrichtung über Fluidleitungen mit einer Manschette verbunden ist, die wenigstens eine Kammer enthält, und wobei eine Oszillationsnachweiseinrichtung mit der oder den Kammern der Manschette verbunden ist.

Bei der üblichen, "unblutigen" Blutdruckmessung wird am Arm der zu untersuchenden Person eine Manschette mit einer deformierbaren, aufblasbaren Kammer befestigt, die mit einer Pumpe, einem Entlüftungsventil mit Entlüftungsdrossel und einem Manometer verbunden ist. Bei der Durchführung der Messung wird die Kammer auf einen über dem zu erwartenden systolischen Druck liegenden Druck aufgepumpt und danach langsam entlüftet. Dabei werden die Korotkoff-Töne abgehört, und die Druckwerte beim Auftreten bzw. Verschwinden dieser Töne werden als der systolische bzw. diastolische Druck bewertet. Abgesehen davon, daß in der medizinischen Wissenschaft keine Übereinstimmung darüber besteht, ob der sogenannte 4. oder 5. Korotkoff-Ton besser den diastolischen Druck kennzeichnet, ist es auch aufgrund entstehender Störgeräusche schwierig, die Druckwerte einwandfrei zu bestimmen.

Die oszillatorische Blutdruckmessung nach von Recklinghausen bedient sich einer Doppelmanschette mit einer proximalen und einer distalen Kammer und beruht darauf, daß der Blutdruckimpuls in der proximalen Kammer in der systolischen Phase vor dem in der distalen Kammer auftritt, während in der diastolischen Phase die Blutdruckimpuls-Phasendifferenz verschwindet. Im Gerät sind eine dickwandige Membrandose zur Anzeige des absoluten Drucks, eine dünnwandige Membrandose zur Wiedergabe der pulsatorischen Druckoszillationen, ein Hebel mit zwei Drehpunkten in Verbindung mit den Membrandosen und ein Umschalter für die Kammern der Doppelmanschette vorgesehen. Gemessen wird bei fallendem Manschettendruck, der mittels einer Ablaufdrossel erzeugt wird. Die Oszillationen in beiden Kammern überlagern sich im Zeigergerät, wobei der Maßstabsfaktor für den Blutdruck verloren geht. Die Bedienungsperson ist darauf angewiesen, die Art des Zeigerausschlags ("Hüpfschritt") zu beurteilen. Dies gelingt nur geschultem Personal.

Man hat deshalb die Oszillationsmethode mit elektronischen Mitteln zu verbessen versucht. Dabei wurden die Blutdruckpulsationen durch Anblasen je eines Thermistors ermittelt, der in einem Verbindungsschlauch zwischen dem Kammern angeordnet ist (DE-A-1 293 392). Absolute Druckmeßwerte können auf diese Weise nicht ermittelt werden.

Während in den zuvor erwähnten Methoden die Ermittlung der Blutdruckpulsationen nur indirekte Bedeutung - als Hilfsmittel bei der Bestimmung des systolischen und diastolischen Druckes - hat, kommt der absoluten Höhe und dem Verlauf der Blutdruckpulsationen diagnostische Bedeutung zu.

Der Erfindung liegt die Aufgabe zugrunde, ein System zur Feststellung von Blutdruckpulsationen zu - schaffen, bei welchem physikalische Größen exakt erfaßt werden und das von subjektiven Beobachtungsfehlern der Bedienungsperson frei ist.

Man geht von einer bestimmten Belastungssituation der untersuchten Person infolge der angelegten Manschette aus und will die Körperreaktion in den Blutdruckpulsationen messen. Mit den auftretenden Druckimpulsen werden Kenngrößen zur Beurteilung der untersuchten Person gewonnen, nämlich Merkmale über die Beschaffenheit der Arterienwandung und dergleichen.

Die gestellte Aufgabe wird dadurch gelöst, daß der Fluiddruck stufenweise über einen Meßbereich anhebbar bzw. absenkbar ist und daß die Oszillationsnachweiseinrichtung aus einer Druckmeßeinrichtung besteht, die den Druck als exakte physikalische Größe zu messen ermöglicht.

Das Pulsationsmeßgerät weist zweckmäßigerweise ein Füllventil zur Verbindung der Fluiddruckerzeu-gungseinrichtung mit der oder den Kammern der Manschette, ferner ein Verbindungsventil zur Verbindung und Abtrennung der beiden Kammern voneinander und ein Belüftungsventil zur Verbindung der einen Seite der Differenzdruckmeßeinrichtung mit der Umgebungsluft bzw. mit einer der Kammern auf. Durch geeignetes Betätigen dieser Ventile kann die Doppelmanschette auf den jeweils einzustellenden Druck gebracht werden, danach werden die beiden Kammern der Manschette fluidmäßig voneinander getrennt und mit den beiden Seiten einer Differenzdruckmeßeinrichtung verbunden. Daraufhin wird die Druckdifferenz zwischen den beiden Kammern und der absolute Wert des Drucks gemessen. Danach wird das System auf ein anderes Druckniveau eingestellt, und die Messungen werden wiederholt. Man kann dies sowohl für stufenweise abfallende Druckwerte als auch für stufenweise zunehmende Druckwerte tun.

Zur automatischen Durchführung der Messungen ist eine Steuerelektronik vorgesehen, welche die - elektromagnetischen - Ventile betätigt und deren Stellung für den Meßablauf steuert.

Zweckmäßigerweise weist die Druckmeßeinrichtung einen piezoelektrischen Wandler als Meßglied auf. Ein solches Meßglied läßt sich einfach mit der Steuerelektronik kombinieren. Die Steuerelektronik kann außerdem eine Programmsteuereinrichtung zur Durchführung mehrerer Meßabläufe und Speichereinrichtungen zur Speicherung der dabei gewonnenen Meßwerte beinhalten. Dabei ist es zweckmäßig, der Steuerelektronik Ausgabegeräte zuzuordnen, um die gemessenen Werte in geeigneter Weise (in Form einer Diskette oder als Graphik) festzuhalten.

Ein Ausführungsbeispiel der Erfindung wird anhand der Zeichnung beschrieben. Dabei zeigt:

Fig. 1 ein Schaltbild eines Pulsationsmeßgeräts,

Fig. 2 ein Blockschaltbild der Steuerelektronik und

Fig. 3 ein Pulsationsdiagramm

Es wird Bezug auf Fig. 1 genommen. Eine Fluiddruckerzeugungseinrichtung 1, die aus einer Handpumpe für Luft, Fußpumpe, Kompressor oder Druckflasche bestehen kann, ist mit einem Drucksammelbehälter 2 verbunden, dessen Druckhöhe durch ein Manometer 3 anzeigbar ist. Am Auslaß des Behälters 2 sitzt ein Füllventil 4, das als elektromagnetisches Wegeventil mit zwei Anschlüssen und zwei bestimmten Stellungen ausgebildet sein kann. Das Ventil 4 dient dazu, Fluiddruck in ein Leitungssystem 5 bis 11 einzulassen, das mit einer Doppelmanschette 12 verbunden ist, die zwei sich teilweise überlappende Kammern 13 und 14 enthält. In der Leitung 6 ist ein Belüftungsventil 15 vorgesehen, das als elektromagnetisch betätigtes Wegeventil mit drei Anschlüssen und zwei bestimmten Stellungen ausgebildet ist. Die Leitung 7 enthält ein Verbindungsventil 16, das als durch Elektromagnete betätigtes Wegeventil mit zwei Anschlüssen und zwei bestimmten Stellungen ausgebildet ist. An die Leitung 5 ist ein Ablaßventil 17 angeschlossen, das zur Belüftung des Systems dient. An der Leitung 5 kann auch ein weiteres Manometer 18 angeschlossen sein, welches zur laufenden Überwachung des Drucks im Leitungssystem 5 bis 11 dient. Schließlich ist noch eine elektronische Druckmeßeinrichtung 20 mit druckbeaufschlagten Seiten 21 und 22 vorgesehen, die an die Leitung 6 bzw. an die Leitung 8 angeschlossen sind. Die elektronische Druckmeßeinrichtung 20 kann mit einem Meßverstärker 23 versehen sein und steht über eine elektrische Leitung 24 mit einer Steuerelektronik 30 in Verbindung. Weitere elektrische Steuerleitungen 25 bis 28 sind zwischen den Ventilen 4, 15, 16, 17 und der Steuerelektronik 30 vorgesehen.

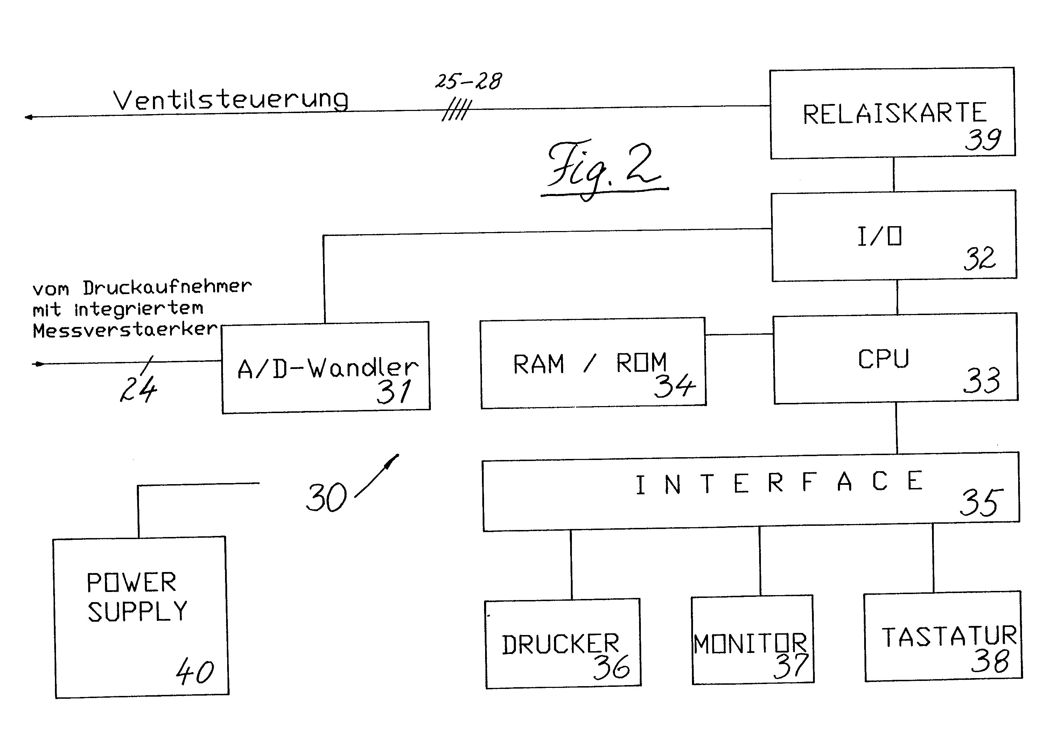

In Fig. 2 ist ein Blockschaltbild als Ausführungsbeispiel der Steuerelektronik 30 dargestellt. Die Eingangsleitung 24 führt zu einem Analogdigitalwandler 31, der mit einer digitalen Eingangs-Ausgangsschaltung 32 verbunden ist. Die digitale Eingangs-Ausgangsschaltung 32 wird von einem Prozessor 33 gesteuert, dem Speichereinheiten 34 zugeordnet sind, welche Schreib-Lese-Speicher und/oder Festwertspeicher enthalten können. Die Eingangs-Ausgangsschaltung 32 ist gegebenenfalls über den zentralen Prozessor 33 noch mit einer Schnittstellenschaltung 35 verbunden, über welche die Verbindung zu einem Drucker 36, einem Monitor 37 und einer Tastatur 38 läuft. Ausgangsseitig ist die Eingangs-Ausgangs-Schaltung 32 mit einer Relaiskarte 39 verbunden, in welcher die Steuerströme für die Ventile 4, 15, 16, 17 gebildet werden. Den dargestellten Baugruppen ist noch eine Stromversorgung 40 zugeordnet.

Die Betriebsweise des Blutdruckpulsationsmeßgeräts ist wie folgt:

Die Doppelmanschette 12 wird um ein Körperteil, vorzugsweise den Oberarm, gelegt. Die Ventile 4 und 16 werden geöffnet, so daß sich ein Fluiddruck in dem Leitungssystem 5 bis 11 aufbaut, der mit dem Manometer 18 überwacht werden kann. Die genaue Einstellung der jeweiligen Belastungsdruckwerte erfolgt mit der Druckmeßeinrichtung 20, deren Seite 21 zu diesem Zweck über das Ventil 15 mit der Umgebungsluft verbunden ist, während die Seite 22 mit dem Fluidleitungssystem 5 bis 11 in Verbindung bleibt. Beim Erreichen des ersten Druckniveaus gibt die Steuerelektronik jeweilige Steuerbefehle über die Leitungen 25, 26, 27, so daß die Ventile 4 und 16 geschlossen und das Ventil 15 auf Verbindung der Druckseite 21 mit der Leitung 6 umgestellt wird. Die distale Kammer 13 ist so fluidmäßig über den Schlauch 10 und die Leitungen 5, 6 und 9 mit der Seite 21 der Druckmeßeinrichtung 20 verbunden, während die proximale Kammer 14 über den Schlauch 11 und die Leitung 8 mit der Seite 22 der Druckmeßeinrichtung 20 in Verbindung bleibt. Die Druckmeßeinrichtung 20 wirkt nunmehr als Differenz druckmesser, d. h. die von dem Körperteil ausgehenden Druckimpulse werden in ihrer Höhe gegenüber dem jeweiligen Belastungsdruckwert gemessen und an die Steuerelektronik 30 weitergereicht, wo sie in entsprechender Form in der Speichereinrichtung 34 festgehalten werden.

Der beschriebene Vorgang wird bei verschiedenen Belastungsdrücken wiederholt. Es wird also insgesamt eine stufenförmige Belastungskurve durchfahren, wobei jede Stufe einem Belastungsdruckwert entspricht. Die Belastungskurve kann ansteigend oder abfallend durchfahren werden. Die Tabelle enthält ein Meßbeispiel, das in Fig. 3 graphisch dargestellt ist. Die ansteigende oder ascendentierende Messung ist mit

3

Punkten, die absteigende oder descendentierende Messung mit Kreuzen gekennzeichnet. In der Elektronik wird noch sie Summer der Abweichungen gegenüber der Belastungskurve, ferner die Differenz zwischen ascendierenden und descendierenden Meßwerten sowie das Abweichungsverhältnis errechnet und ausgegeben.

Wenn die Systole und Diastole bestimmt werden sollen, wird die Belastungskurve mit kleinen Stufen durchfahren, wobei die Stufenhöhe die Genauigkeit der Bestimmung von Systole und Diastole festlegt. Die Systole wird aus einem Formkriterium gewonnen, nämlich dem plötzlichen Übergang der Pulsationen zu hohen Werten. Die Diastole wird zu dem Zeitpunkt bestimmt, wenn die Phasendifferenz zwischen der von der proximalen und der distalen Kammer gewonnenen Druckimpulsen verschwindet. Es versteht sich, daß für solche Bestimmungen entsprechende Elektronik im Bauteil 30 vorgesehen ist.

Als Druckmeßeinrichtung 20 wird ein piezoelektrischer Wandler bevorzugt. Während die Anordnung der Druckmeßeinrichtung zur Differenzdruckmessung bevorzugt wird, ist auch die Anordnung von einzelnen, gegen dem Umgebungsdruck geschalteten piezoelektrischen Elementen möglich. Die Differenzbildung zur Darstellung der Blutdruckimpulse erfolgt dann in der Elektronik.

Das Manometer 18 ist kein notwendiges Teil des Geräts, da der Druck im System von der Druckmeßeinrichtung 20 feststellbar ist. Das Manometer 18 dient dem Wunsch einer stetigen Anzeige des Drucks in der proximalen Manschettenkammer 13. Durch Betätigen entsprechender Tasten in der Tastatur 38 kann die Bedienungsperson die Ventile 16 und 17 öffnen, um den Fluiddruck ganz rasch zu erniedrigen oder abzulassen, wenn dies mit Rücksicht auf den Patienten wünschenswert erscheint.

Wenn man das Belüftungsventil 15 als 3/3-Wegeventil auslegt, kann man eine Schaltstellung mit Verbindung der Fluidleitung 6 zur Atmosphäre schaffen, d.h. man kann dann das Ablaßventil 17 fortlassen, da dessen Funktion von der dritten Stufe des Ventils 15 übernommen wird.

Es versteht wich, daß mehrere solcher Abwandlungen möglich sind, ohne aus dem Bereich der Erfindung zu gelangen.

```
MMHG  -  ASC/DSC

         (■)  (✕)

  10  -   0/  2              130  -  18/  7
  20  -   1/  8              140  -  15/  6
  30  -   3/ 13              150  -   9/  5
  40  -   4/ 21              160  -   7/  5
  50  -  10/ 30              170  -   4/  0
  60  -  16/ 38             180  -   0/  0
  70  -  30/ 41              190  -   0/  0
  80  -  43/ 43              200  -   0/  0
  90  -  49/ 37             _____
 100  -  43/ 29
 110  -  37/ 10             TOTAL:  316/302
 120  -  27/  7
                            A/D:  4%
```

## Ansprüche

1. System zur Feststellung von Blutdruckpulsationen mit folgenden Merkmalen:
eine Fluiddruckerzeugungseinrichtung (1 bis 3) ist über Fluidleitungen (5 bis 11) mit einer Manschette (12) verbunden, die wenigstens eine Kammer (13, 14) enthält;
eine Oszillationsnachweiseinrichtung ist mit der oder den Kammern (13, 14) der Manschette (12) verbunden;
gekennzeichnet durch folgende Maßnahmen:
der Fluiddruck in der Manschette (12) ist als stufenartige Belastungskurve über einen Meßbereich anhebbar bzw. absenkbar;
die Oszillationsnachweiseinrichtung besteht aus einer Druckmeßeinrichtung (20), die den Druck als exakte physikalische Größe zu messen ermöglicht.

2. System nach Anspruch 1,

dadurch gekennzeichnet, daß die Manschette (12) als Doppelmanschette mit einer proximalen Kammer (4) und einer distalen Kammer (13) ausgebildet ist und daß die Druckmeßeinrichtung (20) mit zwei druckbeaufschlagbaren Seiten (21, 22) zur Bildung eines Differenzdruckes ausgebildet ist, wobei sowohl der absolute Druck (gegenüber Umgebungsluft) als auch der relative Druck (zwischen der proximalen und der distalen Kammer) meßbar ist.

3. System nach Anspruch 1 oder 2,

dadurch gekennzeichnet, daß die Druckmeßeinrichtung (20) einen piezoelektrischen Wandler als Meßglied aufweist.

4. System nach einem der Ansprüche 1 bis 3,

dadurch gekennzeichnet, daß in den Fluidleitungen (5 bis 11) einbezogen sind:

ein Füllventil (4) zur Verbindung der Fluiddruckerzeugungseinrichtung (1 bis 3) mit den Kammern (13, 14) der Doppelmanschette (12),

ein Verbindungsventil (16) zur Verbindung und Abtrennung der beiden Kammern (13, 14) voneinander, und

ein Belüftungsventil (15) zur Umschaltung der Druckmeßein richtung (20) auf Messung gegenüber der Umgebungsluft bzw. auf Differenzdruckmessung zwischen den Kammern.

5. System nach einem der Ansprüche 1 bis 4,

dadurch gekennzeichnet, daß eine Steuerelektronik (30) für Meßabläufe vorgesehen ist und daß die bei jedem Meßablauf ermittelten Daten in einem Speicher (34) abspeicherbar sind.

6. System nach Anspruch 5,

dadurch gekennzeichnet, daß die Steuerelektronik (30) Programmsteuereinrichtungen (33) zur Durchführung ascendentierender und descendentierender Meßabläufe aufweise und daß die Programmsteuereinrichtungen (33) zur Errechnung von Meßwertsummen dieser ascendentierenden und descendentierenden Meßabläufe, von Differenzen und von Verhältniswerten dieser Meßabläufe ausgebildet ist.

7. System nach Anspruch 6,

dadurch gekennzeichnet, daß die Steuerelektronik mit einem Ausgabegerät (36, 37) gekoppelt ist, welches Meßwertkurven oder Meßwerttabellen über einen Programmablauf aufzuzeichnen ermöglicht.

8. System nach einem der Ansprüche 5 bis 7,

dadurch gekennzeichnet, daß zur Bestimmung der Systole ein Formkriterium des Meßablaufs ausgewertet wird.

9. System nach einem der Ansprüche 5 bis 8,

dadurch gekennzeichnet, daß zur Bestimmung der Diastole ein Phasenvergleich der Druckimpulse der Kammern (13, 14) der Doppelmanschette erfolgt.

Fig. 1

Blutdruckpulsation

Belastungsdruck [mmHg]

Ventilsteuerung          25-28

Fig. 2

RELAISKARTE 39

I/O 32

vom Druckaufnehmer mit integriertem Messverstaerker

A/D-Wandler 31

RAM / ROM 34

CPU 33

24

30

INTERFACE 35

POWER SUPPLY 40

DRUCKER 36

MONITOR 37

TASTATUR 38

0 266 444

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | WO-A-8 601 704 (BOMED MEDICAL MANUFACTURING LTD.) <br> * Zusammenfassung; Seite 13, Zeile 4 bis Seite 15, Zeile 14; Seite 23, Zeilen 12 bis 20; Seite 24, Zeilen 1 bis 4; Seite 26, Zeile 27 bis Seite 29, Zeile 25; Figuren 1,3,5,6,15 * | 1,2,4-8 | A 61 B 5/02 |
| A | US-A-3 157 177 (P.SMITH) <br> * Spalte 2, Zeile 25 bis Spalte 3, Zeile 32; Figur 1 * | 2-4 | |
| A | US-A-2 452 799 (D.M.SPEAKER et al.) <br> * Spalte 1, Zeile 49 bis Spalte 3, Zeile 11; Spalte 5, Zeilen 3 bis 18; Figur 1 * | 1-4 | |
| A | D.W.HILL: "ELECTRONIC MEASUREMENT TECHNIQUES IN ANAESTHESIA AND SURGERY", 1970, Seiten 76-89, Butterworths & Co., Ltd., London, GB; <br> * Seiten 81,85, Absatz: "Automatic double-cuff methods"; Figuren 2.21 bis 2.23 * | 1,2,4-9 | RECHERCHIERTE SACHGEBIETE (Int. Cl.4) <br><br> A 61 B <br> G 01 L |

Der vorliegende Recherchenbericht wurde für alle Patentansprüche erstellt.

| Recherchenort | Abschlußdatum der Recherche | Prüfer |
|---|---|---|
| DEN HAAG | 08-07-1987 | HUNT, B. W. |